Europäisches Patentamt

(19) European Patent Office

Office européen des brevets

(11) Publication number: **0 109 826**
**A2**

(12) # EUROPEAN PATENT APPLICATION

(21) Application number: **83307000.6**

(51) Int. Cl.³: **A 61 B 5/02**

(22) Date of filing: **16.11.83**

(30) Priority: **17.11.82 US 442460**

(43) Date of publication of application:
**30.05.84 Bulletin 84/22**

(84) Designated Contracting States:
**AT BE CH DE FR GB IT LI LU NL SE**

(71) Applicant: **MEDASID LTD.**
**858 West Jackman**
**Lancaster California 93534(US)**

(72) Inventor: **Scott, David R.**
**44064 North 28th Street**
**Lancaster California 93534(US)**

(72) Inventor: **Stockham, Leo W.**
**7708 Pickard N.E.**
**Albuquerque New Mexico 87112(US)**

(74) Representative: **Stephens, Michael John et al,**
**M.J. Stephens & Co. 46 Tavistock Place**
**Plymouth PL4 8AX(GB)**

(54) **System for deriving medical information.**

(57) A system for deriving medical information by measuring changes (10) in physical size, shape location, weight, etc., of the body or body organs comprises an electro-optical sensor (11) which is either implanted or attached to the body to detect such changes and generate electrical signals (12) responsive thereto, means (13) for conditioning the sensor output signals (12) and means (18) for processing the conditioned signals (16). Provision is made, if required, to correlate the processed signals with external data (17). The medical information (25) is useful in diagnostic, therapeutic or preventive medical procedures.

EP 0 109 826 A2

./...

Croydon Printing Company Ltd.

FIG-1

## SYSTEM FOR DERIVING MEDICAL INFORMATION

This invention relates to a system for deriving medical information which is useful in diagnostic, therapeutic and/or preventive medical procedures.          —

The medical profession's trend toward objective diagnositic and therapeutic  technology has created an increased demand for advanced medical instrumentation systems.  As a result, both the precision of the measurements and the costs of such systems have increased dramatically, resulting in an increased need for instrumentation systems which can make even better measurements at a lower cost.

For example, the stethoscope was the instrument which probably was the most influential in establishing physical examination as a keystone of diagnosis and which started the trend towards objective types of diagnostic therapy. When introduced by Lennec in 1816, the stethoscope merely consisted of a rolled tube of paper, one end of which was placed on the patient's chest and the other in the physician's ear.  The sound is conducted by a column of air.  Frequency response (or filtering) is accomplished by varying the pressure of the stethoscope bell on the skin or by using a plastic diaphragm.  A very recent improvement of the original stethoscope involves the use of an audio amplifier for doctors with deficient hearing or for group teaching. Despite  the marginal evolution of the sensor portion, the stethoscope nevertheless remains a highly effective diagnostic tool in the hands of a skilled physician, as it is responsive, portable, reliable and non-invasive.

However, these same advantages cannot be attributed to many of the modern systems developed for deriving medical information, either due to the high initial cost of the systems or because of operational complexity. Thus, there is a clear need for medical systems useful in diagnosis, therapy and/or preventive medical applications which are less expensive, highly accurate, reliable and which have simplified operational characteristics. A system may meet all of the engineering criteria for its medical use but may not be cost effective because of the need for one or more highly skilled operators and/or the need for the system to be operated under circumstances which do not permit elaborate calibration and/or stabilisation procedures. Although the operation of a medical information derivation system should, ideally, be self-evident to a first-time user, in a more realistic sense, the system should at least be operable by a technician of ordinary skill with only a minimum of easily remembered instructions.

It is, therefore, an object of the invention to provide systems for deriving medical information which have adequate or improved sensitivity, accuracy and reliability, are economic to manufacture and maintain, and can be effectively utilised by personnel of relatively limited medical experience and/or training.

Briefly, in accordance with our invention, these objects are achieved by the provision of a system for deriving medical information from physical changes in at least one of a plurality of body organs, the system comprising electro-optical sensor means for detecting these physical changes and for generating sensor-output electrical signals responsive to these changes; signal-conditioning means for conditioning the sensor output signals, including means for rejecting interfering components thereof; and signal-processing means for processing the conditioned signals, including, if required, correlating the processed signals with external data, to provide said medical information.

Embodiments of the invention can be specially adapted for use in non-invasive procedures while other embodiments can be effectively employed in invasive procedures with improved reliability, sensitivity and safety. The invention is, in particular, applicable to systems for making plethysmographic measurements (such as, for example, velocity or volume of blood flow and changes in size of organs or limbs); to auscultatory systems for deriving cardiovascular information; to systems for deriving orthopedic information; and to systems for deriving other medical information such as fluid intake-outflow (renal) function, in-bed weighing and infant activity and condition.

As used herein, the term "medical information" means and includes information which is useful in diagnostic, therapeutic and/or preventive medical procedures.

As used herein, the term "physical change(s)" means and includes a change or rate of change in any of the physical characteristics of a body organ or group of body organs, such as change or rate of change in dimension(s), shape, weight, location and the like.

As used herein, the term "body organs" means and includes any portion of the human or other animal organism having specialised use or function, such as, without limiting the generality thereof, for example, the brain, heart, eye, ear, nose, tongue, lung, kidney, stomach, intestine, pancreas, gall bladder, liver, penis, womb, endocrine organs, skin, muscles, the skeletal frame including the axial and appendicular skeleton, osseous processes, exo-skeleton and endoskeleton, blood vessels, as well as, in certain applications of the systems of the present invention, all other components including body fluids which contribute to the size, shape or weight of the body or any group of body organs, as defined above.

As used herein, the term "electro-optical sensor" means and includes a sensor device which senses and/or measures the physical changes in a body organ by optical means and which converts the optical indication or measurement to an electrical signal, one or more components of which contain in "raw" form the data which are necessary to yield (after appropriate processing, buffering and, if required, correlation with external data) the desired medical information. By way of illustration and not by way of limitation on the above definition, the electro-optical sensor includes sensors and related peripherals such as those disclosed in issued U.S.A. patents Nos. 4,287,511 and 3,229,511 and in the publication entitled "The Structural Rigidity Sensor: Applications in Non-Destructive Testing," published by the Air Force Systems Command, United States Air Force (Frank J. Seiler Research Laboratory, Publication SRL-TR-75-0017). See also the U.S.A. patents to Okubo Nos. 4,159,422 issued June 26, 1979 , and 4,164,149 issued August 14, 1979. These devices are basically autocollimators which are insensitive to linear dynamic motion but which respond to angular deflection of one end of the sensor with respect to the other and which function by measuring the integral of the structural moment between two points on a structure. In addition, the term "electro-optical sensor" also includes other combinations of light sources, optical components such as lenses, filters and mirrors, and light detectors which are sensitive to linear motion and insensitive to angular deflection or which function to measure other structurally related phenomena, including, for example, angular deflection, angular displacement, strain and shear phenomena.

For any of the end-use applications contemplated herein, the electrical output of the sensor can be fed directly to external peripherals, such as the raw signal-conditioning circuitry and data-processing units. 'Alternatively, and especially where the sensor is used as a permanent or semi-permanent implant by invasive surgical procedures, the signal-conditioning and/or data-processing components of the system can be combined with the sensor in a unitary package for implantation along with suitable means, e.g., a low power RF transmitter, contained within the package to communicate the conditioned and/or processed signal containing the desired medical information to an external receiver for display, recording/editing or control purposes.

As used herein, the term "external input(s)" means and includes data in the form of electrical signals usable in the processing of the sensor output signals or components thereof for correlation with the sensor-derived data to yield the desired medical information. For example, and not by way of limitation, in the case of the use of the system of the invention to monitor fetal heartbeats, the mother's heartbeat, measured by a similar sensor, is utilized along with techniques such as cross-correlation and adaptive noise cancellation to isolate the fetal heartbeat from the background noise. Similarly, and not by way of limitation, in plethysmographic applications involving measurement of circumference of a body organ, the nominal or normal circumference of the organ may be entered as external input to determine medical external input' to determine medical information as to deviation and/or trend of the circumference change.

Systems, embodying the invention, for deriving medical information will now be described, by way of example, with reference to the accompanying diagrammatic drawings, in which:

Fig. 1 is a generalised block flow diagram illustrating the general mode of operation of systems embodying the present invention;

Fig. 2 is a block diagram of a system constituting a presently preferred embodiment of the invention;

Figs. 3 and 4 are electrical schematics which depict, for purposes of illustration, typical circuitry which can be effectively employed in the system of Fig. 2;

Fig. 5 is a sectional view of a structural information detector of the type illustrated in Figs. 2-4, constructed in accordance with a presently preferred embodiment of the invention;

Fig. 6 is a sectional view of a stethoscope bell constructed in accordance with a presently preferred embodiment of the invention, including a generalised blockdiagram of the other major components of the stethoscope system;

Fig. 7 is a perspective view illustrating the use of the stethoscope system of Fig. 6;

Fig. 8 shows an alternate application of the stethoscope sensor of Fig. 6, useful in acoustic monitoring of peripheral cardiovascular phenomenon, in which, in the particular system depicted in Fig. 8, the patient is provided with instantaneous audible information and the information is also simultaneously recorded in a portable support package carried by the patient for later evaluation by medical personell;

Fig. 9 shows yet another application of the stethoscope system of Fig. 6, useful in monitoring and/or recording fetal heartbeat;

Fig. 10 illustrates an embodiment of the invention in which an array of electro-optical sensors is used to perform non-invasive plethysmographic measurements;

Fig. 11 is a sectional view illustrating the presently preferred embodiment of the sensors employed in the system of Fig. 10;

Fig. 12 depicts an implantable plethysmographic system for measuring and/or monitoring the velocity or volume of blood flowing through a major artery or vein of the thigh;

Fig. 13 depicts a system for measuring and/or recording bone characteristics;

Fig. 14 illustrates the use of an embodiment of the present invention for measuring therapeutic orthopedic forces and/or patient weight; and

Fig. 15 illustrates the use of a further embodiment of the present invention for continuously monitoring the activity and physical condition of an infant in its crib, specially adapted for detection and warning if the infant develops Sudden Infant Death Syndrome.

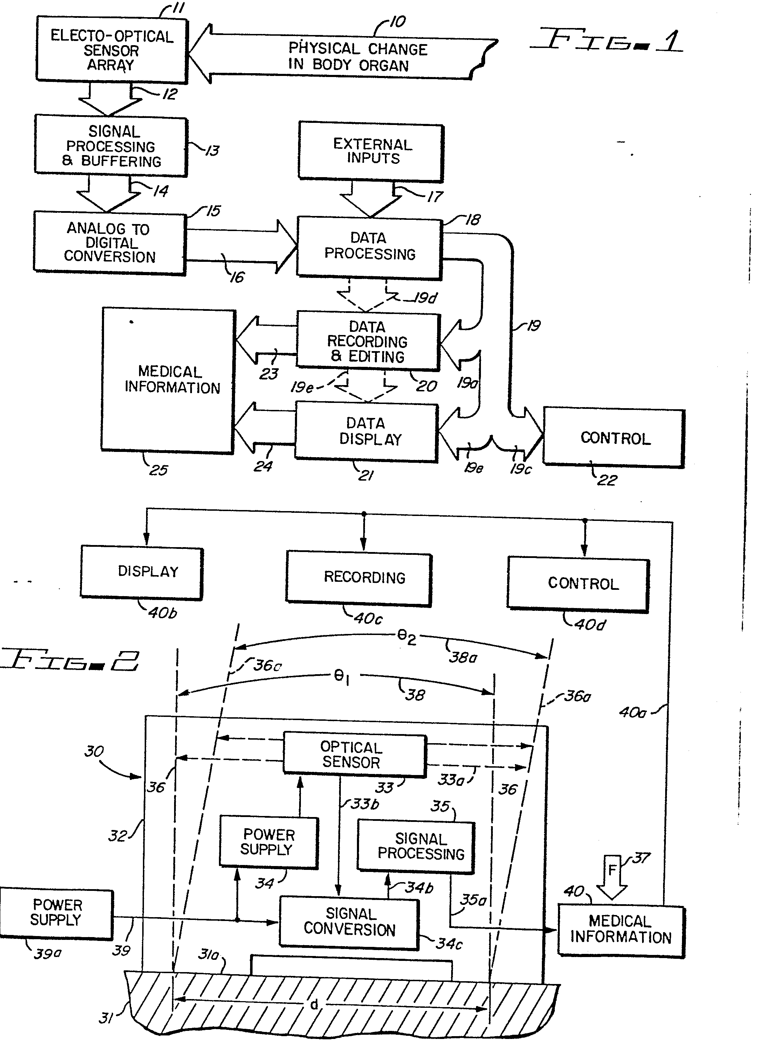

Fig. 1 depicts in block flow diagram form the general mode of operation of systems embodying the invention. As shown in Fig. 1, a physical change in a body organ constitutes an input 10 to an electro-optical sensor or array thereof 11. The sensor output is an electrical signal 12 which is suitably processed and buffered by signal-conditioning means 13 to modify the information content of the signal 12, for example, by rejecting unwanted components or isolating selected components which form the input 14 to analog-to-digital conversion circuitry 16. The digitized signal 16, together with external inputs 17 (e.g., on-off signals, gain, comparative or nominal data), if any, is manipulated by a suitable data-processing computer 18 (e.g. a microprocessor). The output 19 of the data-processing. unit 18, depending on the specific end-use application, forms the input 19a to data-recording and editing units 20, the input 19b to data-display equipment 21 and/or the input 19c to control systems 22. Alternatively, as indicated by the dashed arrows 19d and 19e, the output of the data-processing unit 18 may be fed sequentially to the data-recording and editing unit 20 and to the data-display unit 21, the outputs 23 and 24 of which yield directly usable medical information 25 reflecting the condition, location, etc., of the body organ as inferred or directly measured from the physical changes 10 thereof.

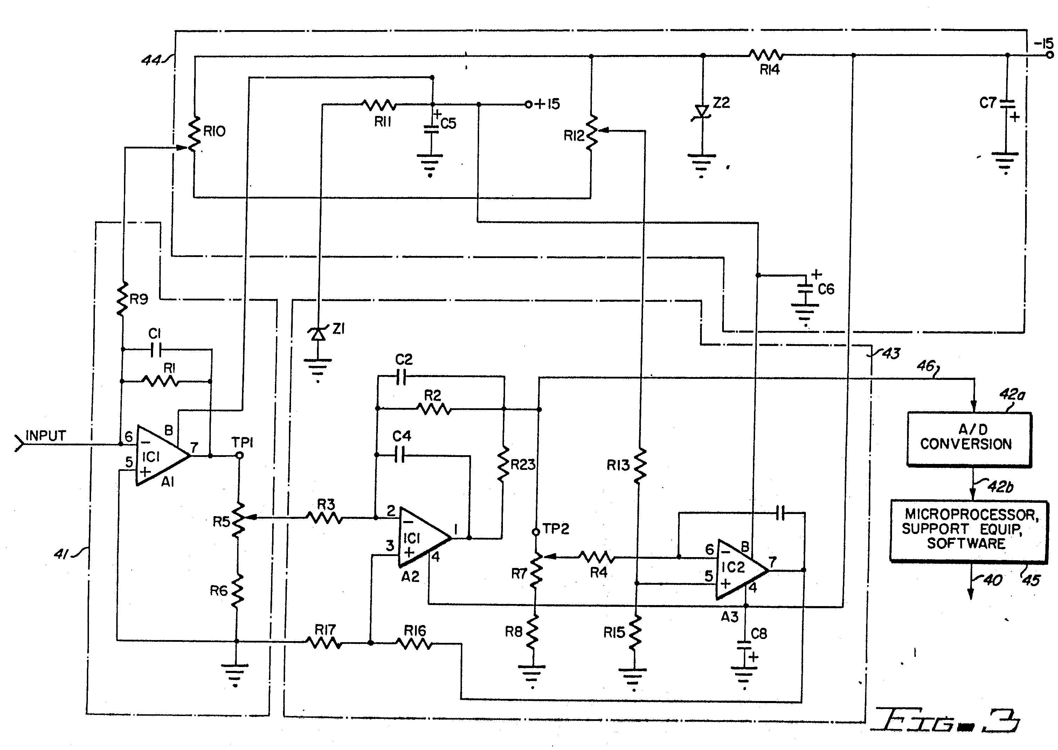

According to the presently preferred embodiment of the present invention, the system of Fig. 1 includes as a major subcomponent, a structural information detector combining the optical sensor of the system with at least certain of the associated electronic signal processing circuits. Figure 2 illustrates the structural information detector 30 in block form, the detector being depicted for purposes of illustration as being mounted upon a support structure 31 which, as explained below, is specially adapted and shaped and/or sized as required by the particular type of medical information to be derived. The structural information detector 30 consists of a housing 32, an optical sensor 33, sensor power supply 34, raw signal conditioning/conversion circuitry 34c and signal processing electronics including a microprocessor and appropriate software 35. The optical sensor 33, preferably a structural moment detector (sometimes also known as a "flexural rigidity sensor") of the type generally disclosed in issued U.S. patent No.' 4,287,511, and in U.S. patent application Serial No. 265,031 measures, as indicated by the dashed line 33a, the relative orientation of surface coordinate vectors which are, as illustratively depicted in Fig. 2, normals 36 to the surface 31a of the beam 31. If a force F (37) is applied in the direction of the arrow 37a to the beam 31, resultant bending of the beam 31 will cause a change in the relative orientation of the surface coordinate vectors 36 to the positions indicated by the dashed lines 36a, i.e., from the angular orientation 38 $\theta_1$ (shown in Fig. 2, illustratively, as $180^{\circ}$) to an angular orientation 38a $\theta_2$ which (as illustratively depicted in Fig. 2) is greater than $\theta1$. Power 39 from an external power source 39a is supplied to the circuitry 34 which, as explained below, provides a regulated power supply to the optical sensor 33. The raw data 33b from the optical sensor 33, which could be a variable voltage or a variable current, is supplied to the raw signal conversion portion 34c of the circuitry, which converts the raw data, as will be further explained below, to a form suitable for feeding to the input 34b of the signal and data-processing electronics and software 35. The unit 35 processes the converted signal 34b and provides, as the output 35a of the structural moment detector 30, a signal which directly indicates the effect of the force F acting on the beam 31 and thereby embodying useful medical information 40. As indicated by the line 40a, the useful information 40 can be utilised in any or all of a variety of ways, i.e., it can be used as the input to a direct display 40b which may be a simple galvanometric meter, liquid crystal display, light emitting diode display, cathode ray tube or the like. Also or alternatively, the useful information 40 can be used as the input to a semi-permanent or permanent recording device 40c, such as paper recorders, magnetic recorders, semiconductor storage devices, optical recorders, bubble memory storage devices, or holographic storage devices. Also or alternatively, the useful information 40 can form the input to various control devices 40d such as servomotors and other similar electromechanical devices.

As will be appreciated by those skilled in the art, and as indicated in issued U.S. patent No. 4,287,511 and U.S. application Serial No. 265,031, it may be necessary or desirable to provide communication paths between some or all of the individual structural information detectors in the array and/or with suitable central data display, recording and/or control components, as well as with additional hardware and software which correlate the useful information outputs of the individual structural moment detectors in the array.

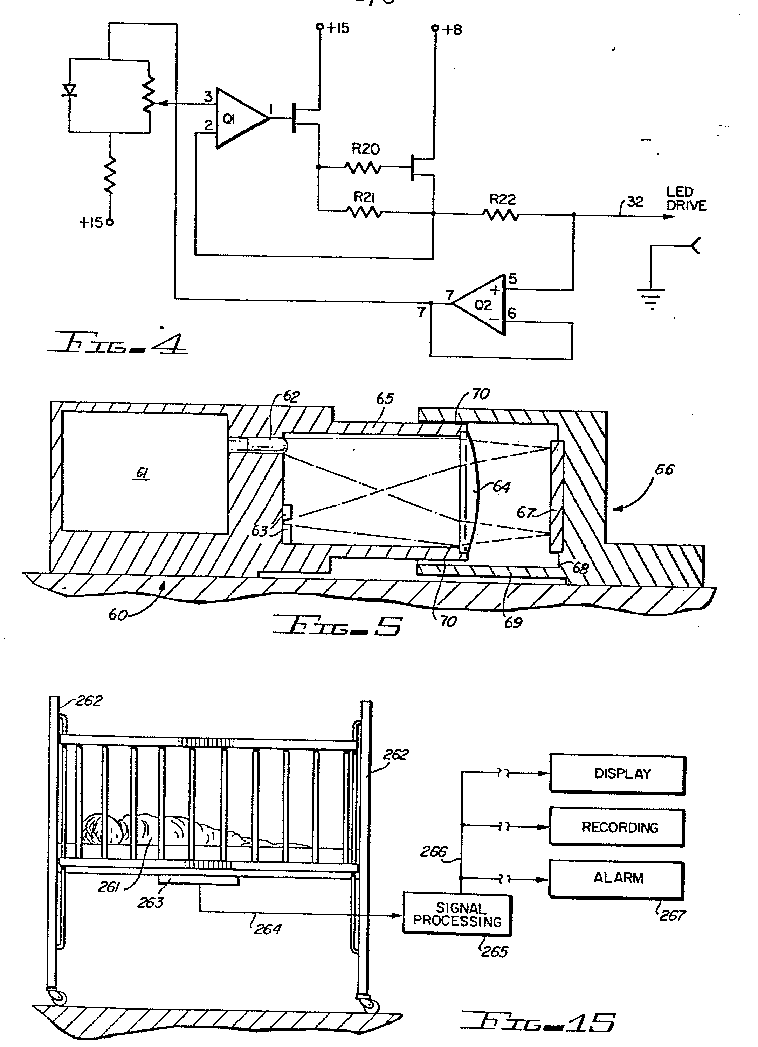

In the presently preferred embodiment of the invention, the raw data output 33b of the optical sensor 33 (Fig. 2) constitutes the input to the signal conversion/ conditioning electronics 41, 43 (Fig. 3). The function of the circuitry 41, 43, depicted in Fig. 3 is to convert and condition the raw data input 33b from the optical sensor into data suitable for electronic data processing and then to feed this data to processing electronics 43 (including computational software) to perform the data-processing function to yield a signal, the components of which directly provide useful medical information. According to the presently preferred embodiment shown in Fig.3, the signal conversion/conditioning is performed with analog electronics, rather than digital. The analog electronics perform two distinct functions. First, the output current into 13b from the optical sensor is converted to a proportional voltage in a transimpedance amplifier circuit which, for clarity of illustration, are those components which are located within the dashed line 41. The second function is to amplify the proportional voltage signal from circuit 41 by feeding it through a gain control 42 into an amplifier which, for purposes of clarity of description, are those components located within the dashed line 43. Circuit 41 operates as a short circuit load for the photovoltaic cells of the optical sensor. Feedback is added in the amplifier circuit 43 to shape the upper end of the frequency response so that spurious high frequency noise is attenuated. This function is performed by applying the output of IC1 (pin 7) to the input (pin 6) of IC2 through a voltage divider formed by R7-R8. IC2 acts as a low-pass filter. The output of IC2 (pin 7) is fed back through R16 to the input 3 of IC1 to act as an automatic bias adjustment. An offset voltage adjustment is provided to remove any bias due to photocell mismatch.

The gain control 42 provides a means of balancing the mechanical gain of different sensors and compensating for components variation. The nominal adjustment range is $\pm15\%$. The amplifier 43 is a high gain direct-coupled amplifier with capacitive feedback to further attenuate high frequency noise levels. Nominal gain is 1.5 volts/microamp and the bandwidth is normally initially set at 50-500 Hz.

Support and bias circuits which include the components which are, for clarity of illustration, enclosed within the dashed line 44, condition power and bias voltages for the components of the circuit 41 and 43.

The output 46 of the amplifier 43 is an analog signal which is converted to a digital signal in the A-D converter 42a. A non-limiting, illustrative, example of a suitable analog-digital converter is manufactured by the U.S. firm Analog Devices under No. HAS1202.

Finally, the signal-processing electronics 45 converts the output signal 42b of the A-D converter 42a into electrical signals 40 which directly provide data related to the effect of the force acting on the structure to which the structural information detector is attached. In the presently preferred embodiment, this component includes a microprocessor, appropriate computational software, the necessary supporting devices and a power supply, details of which are omitted for purposes of clarity because they are well-known to those skilled in the art. Suitable non-limiting examples of microprocessors which can be employed are the TI9000 or the Intel 8086.

Typical non-limiting, illustrative values of the components of Fig. 3 are set forth below:

### Transimpedance Amplifier

R1 - 220K
R5 - 1K
R6 - 2.2K
R9 - 2.2M
R23 - 82
A1 - Operational Amplifier LF353/2

### Second Stage Amplifier

R2 - 680K
R3 - 10K
R4 - 1M

R7  - 100K

R8  - 10K

R13 - 2.2M

R15 - 220K

R16 - 10K

R17 - 1K

C2  - 47PF

C3  - 10MF

C4  - 5PF

A1 - Operational Amplifier LF 353/2

A2 - Operational Amplifier LF 353/2

Support and Bias Circuits

R10 - 100K

R11 - 1.8K

R12 - 100K

R14 - 1.8K

C5  - 10MF

C6  - 10MF

C7  - 10MF

C8  - 10MF

Z1  - LM336

Z2  - LM336

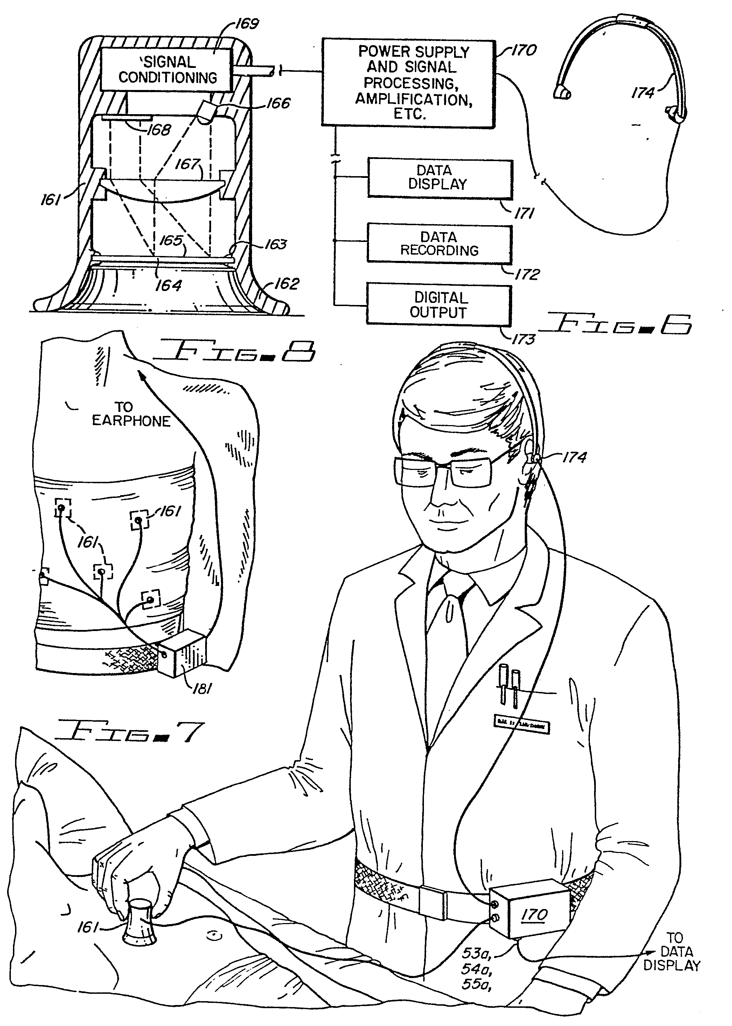

The power supply circuitry 34 (Fig.2) for supplying a precise current to a light emitting diode of the optical sensor 33 is shown schematically in Fig. 4. The 15 volt supply  is provided by the support and bias circuitry 44 of Fig. 3.

The power supply circuitry  of Fig. 4 utilizes two amplifiers in a high gain feedback arrangement to provide the necessary precise current 52 to the light emitting diode.

Typical non-limiting, illustrative values  of the
components of Fig. 4 are set forth below:

Power Supply

R18 - 3.3K
R19 - 10K
R20 - 10K
R21 - 10K
R22 - 47
C9 - 10MF
C10 - 10MF
IC3 - Operational Amplifier LF353/2
Q1 - 2N5457
Q2 - TIP21

All of the elements described in Figs. 3 and 4 may
be fabricated on a printed circuit board using
standard integrated circuits or on a single thick
film substrate where the circuits have been wire
bonded to the substrate.

Although in the Figure 3 arrangement,signal
conditioning functions such as frequency response
tailoring to eliminate unwanted signal components have
been carried out using    analog circuitry, it will be
appreciated that digital techniques  could be used
for this purpose in which case the block 45 would
perform both signal conditioning and signal processing
functions.

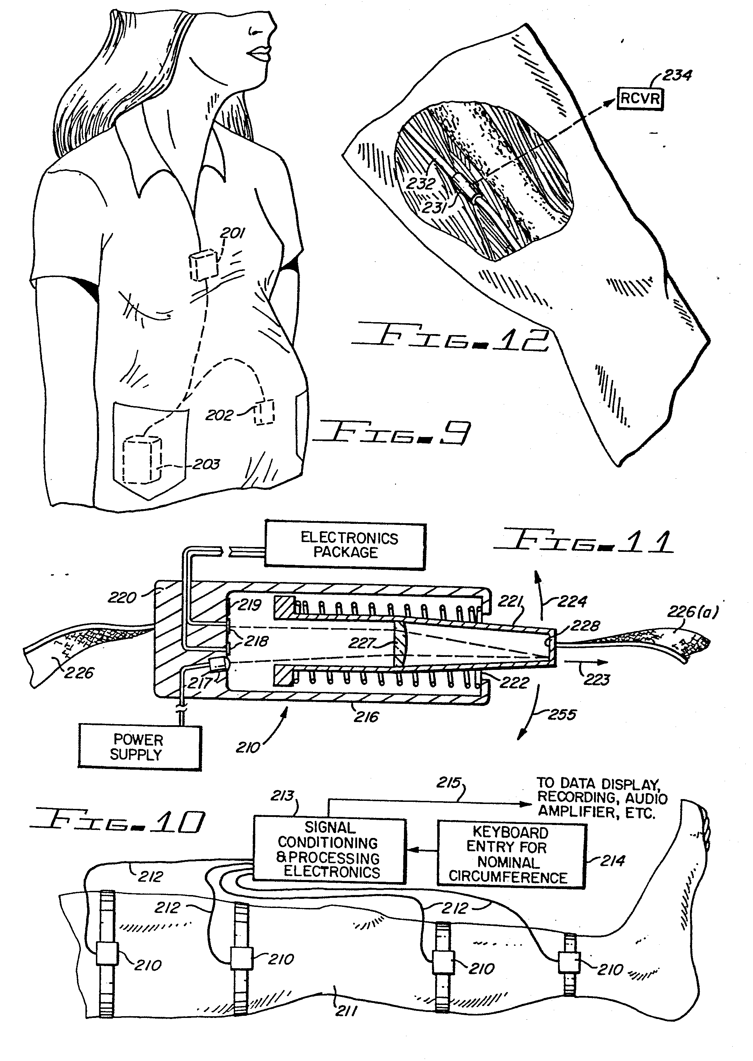

One possible physical form for the structural
information detector (and, in particular, its optical
sensor) is illustrated in Figure 5.  The structural
information detector shown  in Figure 5 consists of a
first housing sub-assembly generally indicated by
reference character 60 containing the sensor power supply/raw signal conversion/signal-processing electronics 61 of Figs. 3-4, a light emitting diode 62, a pair of photovoltaic detectors 63 and a collimating lens 64 carried proximate the open end of a barrel portion 65 formed in the housing sub-assembly 60. A second housing sub-assembly, generally indicated by reference numeral 66, carries a plane surface mirror 67 on the inner end 68 of a mating barrel portion 69 formed in the housing sub-assembly 66. Although a substantial clearance 70 is shown between the barrel portion 65 formed in the first housing sub-assembly 60 and the barrel portion 69 formed in the second housing sub-assembly 66, it will be understood by those skilled in the art that this clearance is shown only for the purpose of clarifying the mechanical relationship of the two housing sub-assemblies 60 and 66. In actuality, the mating barrel portion 69 formed in the second housing sub-assembly 66 is shaped and dimensioned to receive the barrel portion 65 formed in the first housing sub-assembly 60 with an interference fit therebetween, to form a unitary structurally integrated device which excludes ambient light from the interior of the barrel portions 65 and 69 and which facilitates and assists in maintaining precise optical alignment of the two sub-assemblies.

Present work indicates that structural information detectors of the type depicted in Fig. 5 can be successfully manufactured and used which are in the size range of as small as one inch in the major dimension.

In use, the two housing sub-assemblies 60 and 66 are securely fixed to a member at spaced locations therealong so that any bending of this member serves to vary the alignment of the mirror 67 relative to the optical axis of the assembly 60, this variation causing a differential change in the amount of light reaching the detectors 63. The structural information detector shown in Figure 5 is suitable for use in medical-information systems such as will be described hereinafter with reference to Figures 13 to 15.

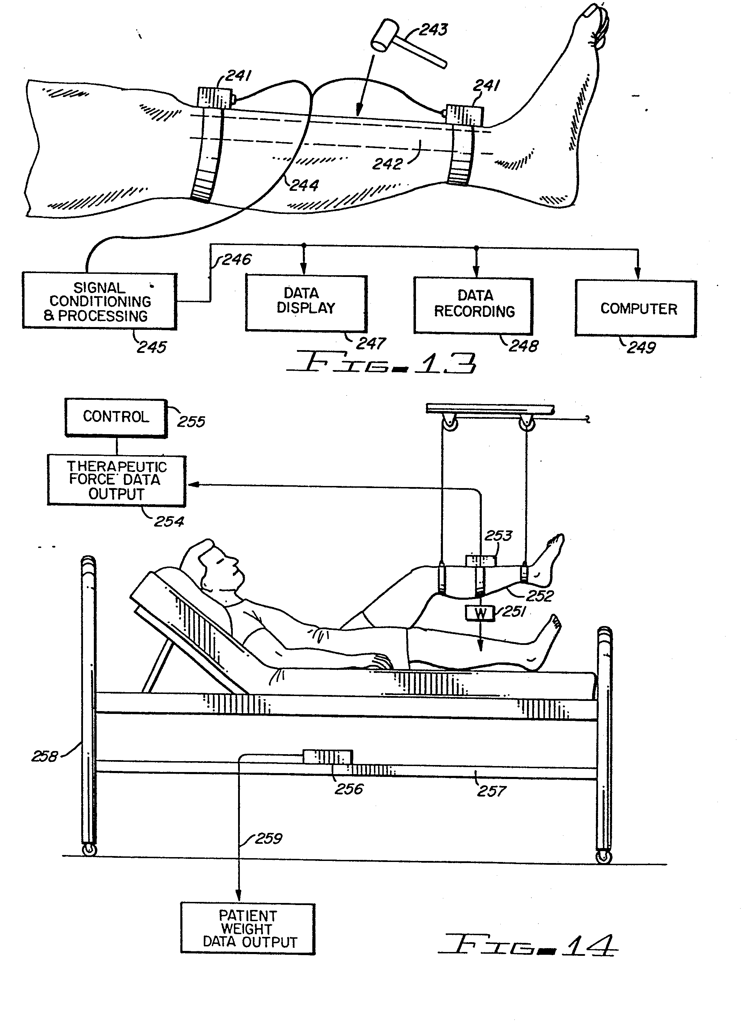

The left hand part of Figure 6 shows a second embodiment of the optical sensor 33, this sensor being constructed as a stethoscope and including a housing portion 161 having a flared open-ended skirt portion 162, shaped and dimensioned to be placed against the patient's body in the manner of a conventional stethoscope bell. An annular internal slot 163 formed in the inner surface of the housing 161 carries a flexible diaphragm 164, the upper surface 165 of which is silvered to be light-reflective. Light emanating from the LED 166 passes through the collimating lens 167 and is reflected as shown by the dashed lines to the photo-voltaic cell 168. In use, the diaphragm 164 is arranged to move in dependence on a physical change being monitored whereby to vary the output of the cell 168. The output of the photovoltaic cell 168 is supplied to signal-conditioning electronics 169. The power supply and signal-processing/amplification electronics 170 furnish outputs to a data display 171, data recording unit 172, a digital output 173, and head phones 174.

Instead of the circular diaphragm mirror depicted in Fig. 6, the stethoscope can be provided with a polygonal mirror, hinged on one side to permit angular displacement of the mirror surface with respect to a pair of photocells in a manner analogous to the sensor of Fig. 5.

A third embodiment of the optical sensor is depicted in cross-section in Fig. 11.  The sensor, generally indicated by the reference character 210, includes a hollow body portion 216 having a light source (LED) 217 and photovoltaic cells 218 carried by the internal face 219 of a closed end 220.  A hollow piston member 221 is received within the hollow portion of the body member 216 and extends through aperture 222 which is sized to permit movement of the piston 221 in the axial direction 223 and in the transverse directions 224 and 225.  Outward axial movement of the member 221 is resisted by a compression spring.  Attachment straps 226 and 226a are affixed, respectively, to the closed end 220 of the body portion 216 and to the outer end of the piston member 221.  Light emitted from the source 217 passes through a collimating lens 227 housed in the piston member 221 and is reflected from the mirrored surface 228 formed on the inner end of the piston member 221.  Light reflected from the mirrored surface 228 passes back through the collimating lens 227 and  falls on the photovoltaic cells 218.   The device functions in a manner analogous to the device of Fig. 5.  Applications of this sensor will be described hereinafter with reference to Figure 10.

Turning now to a consideration of medical end-use applications of systems embodying the invention, the sensor shown in Figure 6 can be used, with the associated electronics  there shown, directly as a stethoscope (see Fig. 7), the power supply and electronics 170 being housed in a belt-mounted package and furnishing outputs via lines 53a, 54a, 55a to the units 171 to 174.

Furthermore, as shown in Fig. 8, an array of the stethoscope bells, configured generally in accordance with Fig. 6, can be semi-permanently attached to a patient's chest to provide semi-continuous phonocardiogram data. If desired, the data-recording unit 172 can be included together with the power supply and the signal-processing/amplification electronics 170, in a belt-mounted package 181 to provide a completely mobile system. The system can also be employed to detect and locate vascular occlusions by locating an array of sensors around the area of the suspected blockage and manipulating the outputs by triangulation techniques to characterise and locate the actual blockage.

As depicted in Fig. 9, the system of the present invention can be utilised to provide semi-continuous monitoring and/or recording of fetal heartbeat. In accordance with this embodiment, separate stethoscope bells 201 and 202, generally configured in accordance with Fig. 6, are placed, respectively, as shown to receive the maternal heartbeat (201) and the fetal heartbeat (202). The maternal heartbeat received from the bell 201 is fed to data processing-recording/editing circuitry contained in an external package 203. The monitored "physical change" (in this case, fetal heartbeat) from the stethoscope bell 202 is also fed to the data processing-recording /editing circuitry contained in the package 203 and manipulated in such fashion as to isolate the fetal heartbeat from the background noise of the mother's heartbeat by known techniques, such as cross-correlation and adaptive noise cancellation.

Fig. 10 illustrates an embodiment of the present invention which performs non-invasive plethysmographic measurements and utilises a plurality of optical sensors 210 of the Figure 11 form. These sensors 210 are attached at spaced points along a portion of the body such as the leg 211. The outputs 212 of the sensors 210 indicate changes in circumference of the body member and are fed to signal-conditioning and processing electronics 213. If desired, nominal circumferences at each of the spaced points can also be entered from a keyboard 214 and manipulated along with the signals 212 to provide data 215 for display, recording, audio amplification, etc.

Other types of plethysmographic measurement such as increase in circumference of blood vessels can also be made, for example, as schematically illustrated in Fig. 12, in which a cuff 231 is surgically implanted around the femoral artery 232. As shown in Fig. 12, the cuff 231 contains not only an electro-optical sensor, but also a miniaturized transmitter which emits radio signals which are received by an external receiver 234. Lead wires are not normally employed in this application to transmit the signal from the cuff 231 to external data processing-signal recording/editing circuitry in order to avoid the risk of infection.

Orthopedic medical information can be derived from physical changes in body member according to the embodiments such as those illustrated in Figs. 13-14.

As shown in Fig. 13, the changes in bone composition and/or strength can be measured and monitored by attaching optical sensors 241 (for example, of the Figure 5 form) at spaced points along a bone, for example, the tibia 242. Striking the bone 242 with a mallot 243 or by some other calibrated method of applying a known force, causes flexure of the bone 242 which is sensed by the electro-optical sensors 241, the outputs 244 of which are fed to signal-conditioning and processing circuitry 245. The data is compared with nominal bone composition and/or strength characteristics or with prior signals to provide the desired medical information 246 which is fed to data-display 247, data-recording 248 or further data-conversion 249 (such as a computer).

As shown in Fig. 14, the amount and effect of a therapeutic force 251 applied to an extremity such as the leg 252 can be measured by an electro-optical sensor 253 which yields therapeutic force data output 254, which may either be displayed or applied to control circuitry 255 to actually vary the therapeutic force 251 to achieve a desired degree of bending of the leg 252.

Yet another application of the system of the present invention is also depicted in Fig. 14. An electro-optical sensor 256 attached to a component, such as the longitudinal frame 257 of a hospital bed 258, yields patient weight data 259 without the necessity of moving the patient, which is especially useful in the case of infirm or severely injured patients. This system can also be used to derive other types of medical data. For example, by continuously monitoring the weight of the patient, fluid intake-output information can be directly and continuously monitored.

Yet another important application of the system of the present invention is illustrated in Fig. 15, which depicts a system for monitoring the activity and vital signs of an infant 261 in a crib 262 which is provided with an electro-optical sensor 263, the output 264 of which is fed to signal conditioning and processing electronics 265. The output 266 of the signal-processing electronics 265 can be displayed and/or recorded and can activate remote alarm systems 267. The objective of this system is to continuously provide and/or record the physical condition of the infant and to give suitable alarms in case of malfunctions of the infant's body systems to detect the symptoms of crib death or Sudden Infant Death Syndrome.

CLAIMS

1. A system for deriving medical information from physical changes in at least one of a plurality of body organs, characterised in that said system comprises:

    (a)  electro-optical sensors means (11;33) for detecting said changes (10;37) and generating sensor-output electrical signals responsive to said changes (12;33b);

    (b)  signal-conditioning means (13;34c) for conditioning said sensor-output signals (12;33b), including means for rejecting unwanted components thereof; and

    (c)  signal-processing means (18; 35) for processing said conditioned signals (16; 34b), including, if required, correlating said processed signals with external data to provide said medical information (25; 40).

2. A system according to Claim 1, wherein said electro-optical sensor means (33) is contained within a housing (60, 66) together with electronic circuitry (61) forming at least part of said signal-conditioning means and signal-processing means (34c; 35), said housing comprising first and second housing sub-assemblies (60, 66); and wherein:

    (a)  said first housing sub-assembly (60) contains said electronic circuitry (61) and components of said optical means (33) including a light source (62), a plurality of photovoltaic detectors (63), and a collimating lens (64); the light source (62) and photovoltaic detectors (63) being carried on the inner face of a hollow barrel portion (65) of said first housing sub-assembly (60) and said collimating lens (64) being carried proximate the open end of said barrel portion (65)';

(b) the second housing sub-assembly (60) contains, as another component of said optical means (33), a plane surface mirror . (67) carried on the inner end (68) of a barrel portion (69) of the second housing sub-assembly (66), the two said housing barrel portions (65, 69) being shaped and dimensioned to mate one within the other with an interference fit therebetween to form a unitary structurally-integrated device; and

(c) said first and second housing sub-assemblies (60, 66) are mountable at spaced points on a body organ.

3. A system according to Claim 1 wherein said electro-optical sensor means (11) is configured as a stethoscope and comprises a housing (161) having a flared open-ended skirt portion (162) shaped and dimensioned to be placed against a patient's body, a flexible or displaceably-mounted mirror member (164) extending across the interior of the skirt portion (162) and provided with an inwardly-directed reflecting surface (165), and a light source (166), collimating lens (167) and light detector (168) disposed within said housing (161) in such a configuration that light from the light source (166) is directed to the detector (168) via the lens (167) and mirror (164) with movement of the latter being arranged to alter the distribution or amount of light incident on the detector (168), the detector (168) being operative to output electrical signals indicative of the light incident thereon.

4. A system according to Claim 1, wherein the electro-optical sensor means (11) is configured for effecting plethysmographic measurements and comprises:

- a hollow body (216) open at one end (222),
- a light source (217) and light detector (218) carried by the internal face (219) of the end of the body opposite said open end (222),
- a hollow piston member (221) open at one end and received within the hollow body (216), with its open end facing the said internal face (219) of the body (216), the piston member (221) extending with play through the open end (222) of the body (216) and being axially movable therewithin, and the internal face of the closed end of the piston member (221) being formed as a reflecting surface (228), and
- a collimating lens (227) housed internally within the piston member (221), the arrangement being such that axial movement of the piston member (221) within the hollow body (216) is effective to vary the distribution or amount of light falling on the light detector (218) from the light source (217) after reflection at said reflecting surface (228), and the detector (218) being operative to output electrical signals indicative of the light incident thereon.

FIG-1

0109826

1/6

ELECTO-OPTICAL SENSOR ARRAY — 11

PHYSICAL CHANGE IN BODY ORGAN — 10

SIGNAL PROCESSING & BUFFERING — 13

EXTERNAL INPUTS

ANALOG TO DIGITAL CONVERSION — 15

DATA PROCESSING — 18

DATA RECORDING & EDITING

MEDICAL INFORMATION — 25

DATA DISPLAY — 21

CONTROL — 22

DISPLAY — 40b

RECORDING — 40c

CONTROL — 40d

FIG-2

OPTICAL SENSOR — 33

POWER SUPPLY — 34

SIGNAL PROCESSING — 35

POWER SUPPLY — 39a

SIGNAL CONVERSION — 34c

MEDICAL INFORMATION — 40

Fig-3

0109826

Fig-4

Fig-5

DISPLAY

RECORDING

ALARM

SIGNAL PROCESSING

Fig-15

*169*
SIGNAL CONDITIONING

*166*

*168*

*167*

*161*

*165*

*164*

*163*

*162*

FIG-8

POWER SUPPLY AND SIGNAL PROCESSING, AMPLIFICATION, ETC. *170*

DATA DISPLAY *171*

DATA RECORDING *172*

DIGITAL OUTPUT *173*

*174*

FIG-6

TO EARPHONE

*161*

*161*

*161*

*181*

FIG-7

*174*

*170*

*53a,*
*54a,*
*55a,*

TO DATA DISPLAY

*161*

0109826

5/6

FIG-12

FIG-9

FIG-11

FIG-10

*Fig-13*

*Fig-14*